# EUROPEAN PATENT APPLICATION

(11) **EP 4 711 001 A1**
(43) Date of publication of application: **18.03.2026**
(21) Application number: 25199641.9
(22) Date of filing: 02.09.2025
(51) Int. Cl.: A61N 5/10, G16H 20/40

(54) **SYSTEMS AND METHODS FOR MODULATION CONTROL IN RADIOTHERAPY PLANS**

(30) Priority: 11.09.2024 US 202418882650
(71) Applicant: Siemens Healthineers International AG, 6312 Steinhausen (CH)
(72) Inventor: HYTÖNEN, Roni, 02130 Espoo (FI); HALKO, Lauri, 00640 Helsinki (FI); NIEMELÄ, Perttu, 02180 Espoo (FI)
(74) Representative: Mathisen & Macara LLP

(57) **Abstract**

Disclosed herein are methods (200) and systems (100) using artificial intelligence models (420) to control the amount of modulation in radiotherapy plans (440). The system (100) may receive (210) parameters defining a plan objective for a radiotherapy treatment plan (440). The system (100) may establish (220) dose distribution parameters for each field of the plan objective. The system (100) may initialize (230) user-defined field regularization factors for each field of the plan objective. The system (100) may initialize (240) plan regularization factors. **In** response to applying the user-defined field regularization factor to a corresponding field of the plan objective and the plan regularization factor to the plan objective, the system (100) may generate (250) a treatment plan objective incorporating weighted components for the plan objective and each field. The system (100) may transmit (260) the treatment plan objective to a radiotherapy treatment planning computer model. The radiotherapy treatment planning computer model may determine the radiotherapy treatment plan (440) based on the treatment plan objective.

## Description

### TECHNICAL FIELD

This application relates generally to generating, training, and operating artificial intelligence (AI) computer models for controlling the amount of modulation in radiotherapy plans.

### BACKGROUND

Radiation therapy (radiation-based therapy or radiotherapy) is a cancer treatment wherein high doses of radiation can kill cancerous cells. The target region of a patient's body that is intended to receive radiation (e.g., a tumor) is referred to as the planning target volume (PTV). The goal is to deliver enough radiation to the PTV to kill the cancerous cells (also referred to herein as a treatment plan or radiation therapy treatment). However, other organs or anatomical regions that are adjacent to or surrounding the PTV can be in the path of radiation beams and can receive enough radiation to be damaged or harmed. These organs, or anatomical regions, are referred to as organs at risk (OARs). Usually, a physician or a radiologist identifies both the PTV and the OARs before planning a treatment plan.

### SUMMARY

In accordance with a first aspect of the invention, there is provided a system, as defined by claim 1.

In accordance with a second aspect of the invention, there is provided a method, as defined by claim 8.

In accordance with a third aspect of the invention, there is provided a non-transitory machine-readable storage medium as defined by claim 12.

Optional features are defined by the dependent claims.

Radiotherapy (RT) is an important step in treating cancer, particularly for patients with inoperable tumors, where the treatment involves prescribed radiation doses delivered over multiple days or treatment fractions. In this regard, accuracy is crucial, as failing to administer enough radiation to the tumor may result in treatment failure, whereas excessive radiation to the surrounding organs can lead to severe side effects. Image-guided radiotherapy (IGRT), using daily imaging techniques, has advanced treatment precision by verifying patient setup before each treatment fraction. However, patients undergoing radiotherapy often experience physiological regression, such as significant weight loss and tumor shrinkage, which can subtly alter patient anatomy and shift or move the PTV and OARs relative to their initial positions. These systematic changes can lead to shifts in patient anatomy, resulting in the underdosing of the PTV and the overdosing of OARs. Since the tumors and the surrounding organs can deform, the patient's positioning can also lead to the underdosing of the tumor and the overdosing of the surrounding organs. These positioning errors, combined with physiological regression, can complicate treatment accuracy.

To maintain therapeutic effectiveness and minimize side effects, frequent updates to the radiotherapy treatment plan (RTTP) are necessary to account for any anatomical changes during the course of treatment. However, the IGRT or treatment planning systems lack the ability to provide periodic replanning, requiring them to undergo a complex procedure. For instance, replanning involves time-consuming steps such as rescheduling CT scans, recontouring structures, developing new treatment plans, and conducting extensive quality assurance checks. This multistep process can significantly delay treatment delivery, impacting patient care and workflow efficiency in high-volume clinics where maintaining treatment continuity is important.

Furthermore, RTTP creation is typically based on the optimization of certain dosimetric parameters, such as "target coverage," "max dose in the spinal cord," and/or "mean dose in the parotid gland." The used dosimetric parameters and the desired goal values are often based on previously performed outcome studies where there is a correlation between these dosimetric parameters and certain clinical endpoints. The observed correlation between dose distribution and clinical outcomes is sometimes referred to as the "clinical models." Conventional plan optimizers may optimize the RTTP against a given clinical model by defining the desired objectives, but they are bound to use certain generic dosimetric parameters, which were implemented and sometimes hardcoded within the software algorithm during the product development (such as "Dose-to-Volume," "Volume-to-Dose," and/or "Generalized Equivalent Uniform Dose." This static approach is also undesirable because it is time-consuming and computationally intensive to revise the software code.

In the context of multi-field optimized intensity-modulated proton therapy (MFO-IMPT), achieving optimal dose distributions involves careful balancing of conflicting objectives. For example, precise dose delivery to the target volume desires highly modulated dose distributions for individual fields. This modulation may be achieved by varying the intensity of the proton beam across each field. However, excessive modulation within individual fields increases sensitivity to patient movement, which may lead to suboptimal dose delivery and an increased risk of adverse effects. Conversely, reducing field modulation to improve robustness may compromise target dose conformity and overall treatment efficacy. As a result, the optimization problems become particularly complex when considering the interplay between multiple radiation fields and the overall RTTP.

Moreover, traditional approaches to mitigating field modulation in intensity-modulated proton therapy (IMPT) have inherent limitations. For example, single-field optimization (SFO) may independently optimize each radiation field. While this optimization may effectively reduce modulation at the field level, it compromises the global optimization of the treatment plan. As a result, SFO may often yield suboptimal dose distributions in complex treatment cases due to its inability to account for the interplay between different fields. For example, a user may have no direct control over the total planned dose of critical organs. This limitation also limits its applicability to simpler treatment cases.

Alternatively, user-defined field and plan objectives provide a degree of control over individual field contributions and the overall plan dose distribution. However, this approach may introduce significant complexity into the planning process. For example, users may be burdened with defining multiple, often partially overlapping objectives, switching between the plan and individual fields. As a result, managing the interplay between these objectives becomes challenging and time-consuming, thereby hindering efficient treatment plan generation.

To address the challenges of balancing dose conformity and field modulation in MFO-IMPT, the technical solutions described herein can implement a hybrid optimization framework that integrates elements of MFO and SFO to provide flexible control over field modulation. The system can implement an MFO-SFO hybrid optimization algorithm that integrates the strengths of both MFO and SFO techniques. For example, the system can convert target structures' MFO objectives into a weighted MFO-SFO objective pair. As a result, the technical solutions can dynamically adjust the level of coupling between individual fields and the overall plan, allowing for a smooth transition between field-independent (SFO-like) and fully coupled (MFO-like) optimization modes.

Moreover, to provide greater control over the trade-off between dose conformity and field modulation, the technical solutions can incorporate a user-definable modulation parameter. The user-definable modulation parameter can allow clinicians to specify the desired level of field modulation. For example, by directly influencing the optimization algorithm's behavior, the technical solutions can provide a dynamic balance between achieving optimal target coverage and minimizing field-related side effects. Furthermore, to enhance treatment plan optimization and user interaction, the technical solution can incorporate an interactive visualization and feedback platform. The interactive visualization and feedback platform can allow clinicians to dynamically observe the impact of modulation level changes on critical plan quality metrics, including dose-volume histograms (DVHs), target coverage, and organ at risk (OAR) sparing, among others. By providing real-time or near real-time feedback on the trade-offs between dose conformity and field modulation, the technical solutions can provide iterative refining of the RTTP until optimal outcomes are achieved. As a result, the technical solutions described herein can optimize dose distribution while maintaining acceptable levels of field modulation, thereby improving RTTP.

The methods and systems discussed herein provide a solution to improve dose distribution in radiation therapy planning. As discussed herein, uneven dose distribution can occur when optimizing multiple radiation fields together. In multi-field optimization, while the overall dose distribution may be uniform, the individual doses from each field can be highly uneven. This unevenness can lead to problems, especially if the patient moves slightly between field deliveries, resulting in "cold spots" or "hot spots" in the target area.

The methods and systems discussed herein introduce a regularization parameter within the optimization process. This parameter allows the optimization algorithm to not only focus on the overall plan objectives but also ensure a more even dose distribution within each individual field. The regularization can be adjusted by the user, ranging from 0% (no regularization, where the focus is solely on the overall plan) to 100% (where the focus is entirely on achieving even dose distribution within each field).

The methods and systems discussed herein offer a balance between multi-field and single-field optimization, allowing for better target coverage and reducing the risk of uneven dose distribution without significantly compromising the dose to organs at risk. This approach also simplifies the planning process by automating the distribution of plan objectives across individual fields based on the regularization parameter set by the user.

### System

At least one aspect of the technical solutions is directed to a system of controlling the amount of modulation in radiotherapy plans. The system may include one or more processors configured to: receive a plurality of parameters defining a plan objective for a radiotherapy treatment plan; establish dose distribution parameters for each field of the plan objective; initialize one or more user-defined field regularization factors for each field of the plan objective; initialize one or more plan regularization factors, wherein the plan regularization factor is based on at least one of the user-defined field regularization factor or user-defined parameters; in response to applying the user-defined field regularization factor to a corresponding field of the plan objective and the plan regularization factor to the plan objective, generate a treatment plan objective incorporating weighted components for the plan objective and each field of the plan objective; and transmit the treatment plan objective to a radiotherapy treatment planning computer model, the radiotherapy treatment planning computer model configured to determine the radiotherapy treatment plan based on the treatment plan objective.

The plurality of parameters may include at least one of a structure, dose, volume, or weight. The system may initialize values for the plan regularization factor and the user-defined field regularization factor. The system may calculate the weighted component for the plan objective in response to applying the plan regularization factor to the plan objective. The system may calculate the weighted component for the corresponding field of the plan objective in response to applying the user-defined field regularization factor to the corresponding field of the plan objective. The system may provide a control interface configured to receive user interactions for adjusting the user-defined regularization factors. The system may implement the plan objective as a multi-field optimization formulation responsive to identifying that the user-defined field regularization factors are set to a value of zero. The system may implement the plan objective as a full single field optimization coverage of planning target volume responsive to identifying that the user-defined field regularization factors and the plan regularization factor are set to a value of one.

### Method

At least one aspect of the technical solutions is directed to a method of planning or replanning radiotherapy treatment. The method may include: receiving, by at least one processor, a plurality of parameters defining a plan objective for a radiotherapy treatment plan; establishing, by the at least one processor, dose distribution parameters for each field of the plan objective; initializing, by the at least one processor, one or more user-defined field regularization factors for each field of the plan objective; initializing, by the at least one processor, one or more plan regularization factors, wherein the plan regularization factor is based on at least one of the user-defined field regularization factor or user-defined parameters; in response to applying the user-defined field regularization factor to a corresponding field of the plan objective and the plan regularization factor to the plan objective, generating, by the at least one processor, a treatment plan objective incorporating weighted components for the plan objective and each field of the plan objective; and transmitting, by the at least one processor, the treatment plan objective to a radiotherapy treatment planning computer model, the radiotherapy treatment planning computer model configured to determine the radiotherapy treatment plan based on the treatment plan objective.

The plurality of parameters may include at least one of a structure, dose, volume, or weight. The method may include initializing, by the at least one processor, values for the plan regularization factor and the user-defined field regularization factor. The method may include calculating, by the at least one processor, the weighted component for the plan objective in response to applying the plan regularization factor to the plan objective. The method may include calculating, by the at least one processor, the weighted component for the corresponding field of the plan objective in response to applying the user-defined field regularization factor to the corresponding field of the plan objective. The method may include providing, by the least one processor, a control interface configured to receive user interactions for adjusting the user-defined regularization factors.

Optionally, the method further comprises implementing the plan objective as a multi-field optimization formulation responsive to identifying that the user-defined field regularization factors are set to a value of zero.

Optionally, the method further comprises implementing the plan objective as a full single field optimization coverage of planning target volume responsive to identifying that the user-defined field regularization factors and the plan regularization factor are set to a value of one.

### Medium

Yet another aspect of the technical solutions is directed to a non-transitory machine-readable storage medium having computer-executable instructions stored thereon that, when executed by one or more processors, cause the one or more processors to: receive a plurality of parameters defining a plan objective for a radiotherapy treatment plan; establish dose distribution parameters for each field of the plan objective; initialize one or more user-defined field regularization factors for each field of the plan objective; initialize one or more plan regularization factors, wherein the plan regularization factor is based on at least one of the user-defined field regularization factor or user-defined parameters; in response to applying the user-defined field regularization factor to a corresponding field of the plan objective and the plan regularization factor to the plan objective, generate a treatment plan objective incorporating weighted components for the plan objective and each field of the plan objective; and transmit the treatment plan objective to a radiotherapy treatment planning computer model, the radiotherapy treatment planning computer model configured to determine the radiotherapy treatment plan based on the treatment plan objective.

The plurality of parameters may include at least one of a structure, dose, volume, or weight. The instructions may cause the one or more processors to initialize values for the plan regularization factor and the user-defined field regularization factor. The instructions may cause the one or more processors to calculate the weighted component for the plan objective in response to applying the plan regularization factor to the plan objective. The instructions may cause the one or more processors to calculate the weighted component for the corresponding field of the plan objective in response to applying the user-defined field regularization factor to the corresponding field of the plan objective. The instructions may cause the one or more processors to provide a control interface configured to receive user interactions for adjusting the user-defined regularization factors.

Optionally, the instructions cause the one or more processors to implement the plan objective as a multi-field optimization formulation responsive to identifying that the user-defined field regularization factors are set to a value of zero.

Optionally, the instructions cause the one or more processors to implement the plan objective as a full single field optimization coverage of planning target volume responsive to identifying that the user-defined field regularization factors and the plan regularization factor are set to a value of one.

### Key features

Optionally, the present method, system and medium provide a hybrid optimization framework that integrates elements of multi-field optimization and single-field optimization.

Optionally, the present method, system and medium can convert plan objectives into a weighted MFO-SFO objective pair.

Optionally, the present method, system and medium provide a balance between multi-field and single-field optimization.

Optionally, the present method, system and medium balance the conflicting objectives of achieving dose conformity while minimizing field modulation.

Optionally, in the present method, system and medium, the regularization factor may set the extent to which overall plan objectives are achieved, and the extent to which uniform dose distribution is achieved within each field.

Optionally, the method, system and medium may provide a graphical user interface to allow a user to control the balance between plan-level optimization and field-level uniformity.

Optionally, the method, system and medium implement the plan objective as hybrid multi-field optimization and single-field optimization responsive to identifying that the regularization factors are set to a value of between zero and one.

### BRIEF DESCRIPTION OF THE DRAWINGS

Non-limiting embodiments of the present disclosure are described by way of example with reference to the accompanying figures, which are schematic and are not intended to be drawn to scale. Unless indicated as representing the background art, the figures represent aspects of the disclosure.
**FIG. 1** illustrates components of an AI-enabled RTTP generation system, according to an embodiment.
**FIG. 2** illustrates a flow diagram of a process executed in an AI-enabled RTTP generation system, according to an embodiment.
**FIGS. 3A-3G** illustrate an example user interface for optimizing radiotherapy treatment plans, according to an embodiment.
**FIG. 4** illustrates different flow diagrams of different processes executed in an AI-enabled RTTP generation system to predict systematic changes in a patient's anatomy from one treatment fraction to another, according to an embodiment.

### DETAILED DESCRIPTION

Reference will now be made to the illustrative embodiments depicted in the drawings, and specific language will be used herein to describe the same. It will nevertheless be understood that no limitation of the scope of the claims or this disclosure is thereby intended. Alterations and further modifications of the inventive features illustrated herein, and additional applications of the principles of the subject matter illustrated herein that would occur to one skilled in the relevant art and having possession of this disclosure, are to be considered within the scope of the subject matter disclosed herein. Other embodiments may be used and/or other changes may be made without departing from the scope of the present disclosure. The illustrative embodiments described in the detailed description are not meant to be limiting to the subject matter presented.

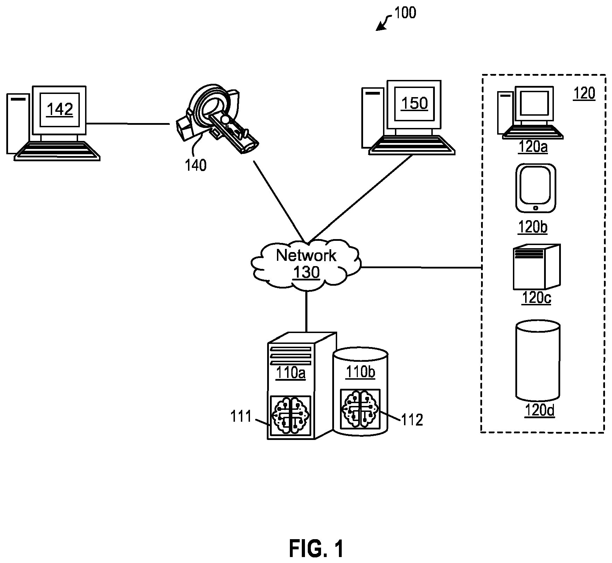

**FIG. 1** illustrates components of an AI-enabled RTTP generation system (system **100),** according to an embodiment. The system **100** may include an analytics server **110a,** system database **110b,** clinical model analysis computer model **111,** end-user devices **120a-d** (collectively end-user devices **120),** and an administrator computing device **150.** Various components depicted in **FIG. 1** may belong to a radiation therapy clinic at which patients may receive radiation therapy treatment via one or more radiation therapy machines (e.g., medical device **140)** in the clinic. The above-mentioned components may be connected through a network **130.** Examples of the network **130** may include, but are not limited to, private or public LAN, WLAN, MAN, WAN, and the Internet. The network **130** may employ wired and/or wireless communications according to one or more standards and/or via one or more transport mediums.

The communication over the network **130** may be performed in accordance with various communication protocols such as Transmission Control Protocol and Internet Protocol (TCP/IP), User Datagram Protocol (UDP), and IEEE communication protocols. In one example, the network **130** may include wireless communications according to Bluetooth specification sets or another standard or proprietary wireless communication protocol. In another example, the network **130** may also include communications over a cellular network, such as a GSM (Global System for Mobile Communications), CDMA (Code Division Multiple Access), or EDGE (Enhanced Data for Global Evolution) network.

The system **100** is not confined to the components described herein and may include additional or other components, not shown for brevity, which is to be considered within the scope of the embodiments described herein.

The analytics server **110a** may generate and display an electronic platform configured to use various computer models **111-112** to identify attributes of an RTTP for a patient's treatment. The electronic platform may include a graphical user interface (GUI) displayed on the end-user devices **120** and/or the administrator computing device **150.** An example of the electronic platform generated and hosted by the analytics server **110a** may be a web-based application or a website configured to be displayed on different types of electronic devices, such as mobile devices, tablets, personal computers, and the like.

In a non-limiting example, a physician operating the physician device **120b** may access the platform, input patient attributes, characteristics, and other data, and further instruct the analytics server **110a** to generate an RTTP (including the beam angle, physiological regression, patient positioning, dose constraints, treatment schedule, etc.). Additionally, or alternatively, the analytics server **110a** may optimize only one particular attribute of the RTTP (e.g., optimize beam angle or dose constraints delivered to the tumor and surrounding healthy organs).

The analytics server **110a** may recommend the RTTP (e.g., beam angles and other radiation parameters and/or treatment plan attributes) used for proton radiation, photon radiation, and electron radiation. In particular, analytics server **110a** may utilize the methods and systems described herein to execute the clinical model analysis computer **111** for identifying cost values or predicting anatomical changes in the patient's anatomy that can ultimately be used (e.g., by the plan optimizer model **112)** to evaluate and re-evaluate the RTTP. Further, the analytics server **110a** may transmit the beam angles and other radiation parameters and/or treatment plan attributes to one or more other servers. Additionally, or alternatively, the analytics server **110a** (or another server) may adjust the configuration of one or more devices (e.g., the medical device **140)** based on the systematic changes observed in the patient's anatomy from one treatment fraction to another. For instance, the RTTP may be directly sent to the medical device **140** and/or the computer **142** that functionally controls the medical device **140** used in the treatment plan. For instance, the optimized RTTP, including beam angles, monitor units (MU), and other treatment parameters, may be directly transmitted to the medical device **140** or its controlling computer **142** for implementation.

The medical device **140** may be any medical device used in the radiation therapy treatment of a patient (such as a CT scan machine, radiation therapy machine (e.g., a linear accelerator, particle accelerator (including circular accelerators), or cobalt machine)). The medical device **140** may also include an imaging device capable of emitting radiation such that the medical device **140** may obtain images according to various methods to accurately image the internal anatomical structure of a patient. For instance, the medical device **140** may include a rotating system (e.g., a static or rotating multi-view system). A non-limiting example of a multi-view system may include stereo systems (e.g., two systems arranged orthogonally).

The analytics server **110a** may host a website accessible to users operating any of the electronic devices described herein (e.g., end-users or medical professionals), where the content presented via the various webpages may be controlled based upon each particular user's role or viewing permissions. The analytics server **110a** may be any computing device comprising a processor and non-transitory, machine-readable storage capable of executing the various tasks and processes described herein. The analytics server **110a** may employ various processors, such as central processing units (CPU) and graphics processing units (GPU), among others. Non-limiting examples of such computing devices may include workstation computers, laptop computers, server computers, and the like. While the system **100** includes a single analytics server **110a,** the analytics server **110a** may include any number of computing devices operating in a distributed computing environment, such as a cloud environment.

The analytics server **110a** may execute software applications configured to display the electronic platform (e.g., host a website), which may generate and serve various webpages to each end-user devices **120.** Different users may use the website to view and/or interact with the recommended (optimized) results. Different servers, such as a clinic server **120c,** may also use the recommended results in downstream processing.

The analytics server **110a** may be configured to require user authentication based upon a set of user authorization credentials (e.g., username, password, biometrics, cryptographic certificate, and the like). The analytics server **110a** may access the system database **110b** configured to store user credentials, which the analytics server **110a** may be configured to reference in order to determine whether a set of entered credentials (purportedly identifying the user) match an appropriate set of credentials that authenticate the user.

The analytics server **110a** may generate and host webpages based upon a particular user's role within the system **100.** In such implementations, the user's role may be defined by data fields and input fields in user records stored in the system database **110b.** The analytics server **110a** may authenticate the user and may identify the user's role by executing an access directory protocol (e.g., LDAP). The analytics server **110a** may generate webpage content that is customized according to the user's role as defined by the user record in the system database **110b.**

The analytics server **110a** may receive various clinical objectives, patient data, and treatment data from the end-user devices **120.** For instance, a physician may access the platform provided by the analytics server **110a** using a physician device **120b.** The physician may input various patient attributes and/or clinical objectives using one or more input elements of the platform. For instance, a physician may input patient attributes and clinical objectives, such as target volumes, organs at risk, dose constraints, and treatment modality (proton, photon, or electron), into the platform provided by the analytics server **110a** using a physician device **120b.** The analytics server **110a** may then execute various methods discussed herein, including optimization algorithms (e.g., SFO, MFO) and regularization techniques, and display an RTTP on the platform.

The end-user devices **120** may be any computing device comprising a processor and a non-transitory machine-readable storage medium capable of performing the various tasks and processes described herein. Non-limiting examples of end-user devices **120** may be a workstation computer, laptop computer, tablet computer, and server computer. During operation, various users may use end-user devices **120** to access the GUI operated by the analytics server **110a.** Specifically, the end-user devices **120** may include a clinic computer **120a,** a physician device **120b,** a clinic server **120c,** or a clinic database **120d.**

In order to generate the RTTP, the analytics server **110a** may then execute various models (e.g., clinical model analysis computer model **111** and/or a plan optimizer model **112)** to analyze the retrieved/received data.

The administrator computing device **150** may represent a computing device operated by a system administrator. The administrator computing device **150** may be configured to display beam angles, radiation parameters, and/or other radiation therapy treatment attributes generated by the analytics server **110a;** monitor the clinical model analysis computer model **111** utilized by the analytics server **110a** and/or end-user devices **120;** review feedback; and/or facilitate training or retraining (calibration) of the models **111-112** that are maintained by the analytics server **110a.**

The analytics server **110a** may be in communication (real-time or near real-time) with the medical device **140** (or its computer **142),** such that a server/computer hosting the medical device **140** may adjust the medical device **140** based on the RTTP (e.g., beam angles, MU, optimization algorithms (e.g., SFO, MFO), regularization techniques, treatment attributes, and/or other radiation parameters determined by the analytics server **110a).** For instance, the radiation therapy machine may adjust the gantry, beam blocking device (e.g., multi-leaf collimator MLC), and couch setup based on dose constraints or optimized beam angles, where the optimized beam angle is an angle of the medical device **140** that emits radiation in a direction of the PTVs. The analytics server **110a** may transmit instructions to the radiation therapy machines indicating any number or type of radiation parameters and/or treatment attributes to facilitate such adjustments.

The clinical model analysis computer model **111** and/or **112** may represent any collection of algorithmic logic and/or artificial intelligence models (e.g., using various machine learning techniques). In some embodiments, the clinical model analysis computer model **111** and/or **112** may include a convolutional long short-term memory (LSTM) model. The clinical model analysis computer model **111** may include various algorithms to identify a cost value for an RTTP based on patient data and/or treatment attributes and how they would likely cause a secondary and/or long-term effect (e.g., health problem). In some embodiments, the clinical model analysis computer model **111** may include various algorithms that process a series of treatment fraction images and predict anatomical changes in at least one structure of the patient for a forecasted treatment fraction. Using the clinical model analysis computer model **111,** the analytics server **110a** may execute the plan optimizer model **112.** The plan optimizer model **112** may iteratively refine the treatment plan by adjusting parameters such as beam angles, MU, field weights, and regularization factors, among others. The plan optimizer model 112 may optimize the treatment plan by balancing the competing objectives of minimizing field modulation and achieving adequate target coverage. In some embodiments, to improve the treatment plan, the analytics server **110a** may integrate insights from the clinical model analysis, such as predicted anatomical changes, cost-effectiveness metrics, or plan objectives.

The analytics server **110a** may execute the models in conjunction with each other and then revise one or more configuration or treatment parameters of the plan optimizer **112.** In some embodiments, the analytics server **110a** may execute the plan optimizer model **112** in conjunction with other models to revise the initial treatment plan in response to determining that at least one structure of the patient is being underdosed or overdosed. As a result, the efficiencies discussed herein can be achieved without revising the plan optimizer **112** itself. For instance, the clinical model analysis computer model **111** can be executed independently and used in conjunction with an existing plan optimizer. Therefore, the plan optimizer (or the system infrastructure) can be retrofitted using the methods and systems discussed herein. This minimal interference with existing infrastructure allows for the improvement of a plan optimizer without the need to revise its source code.

In some embodiments, the analytics server **110a** may collect patient data from various sources not shown in **FIG. 1****.** For instance, the analytics server **110a** may monitor and collect patient and treatment attributes associated with previously-treated radiotherapy patients to include in a training dataset. This may include data on the number of treatment fractions undergone by each patient, treatment details, treatment outcomes, and other relevant attributes. Additionally, or alternatively, the analytics server **110a** may collect/aggregate other patient data (e.g., data associated with how a set of patients or clinical participants received their respective radiotherapy treatment). Using the collected data, the analytics server **110a** may generate a training dataset with which the analytics server **110a** may train the clinical model analysis computer model **111** to identify patterns, predict outcomes, and optimize treatment plans. As a result, the clinical model analysis computer model **111** may be used to limit the search space used by the plan optimizer **112.**

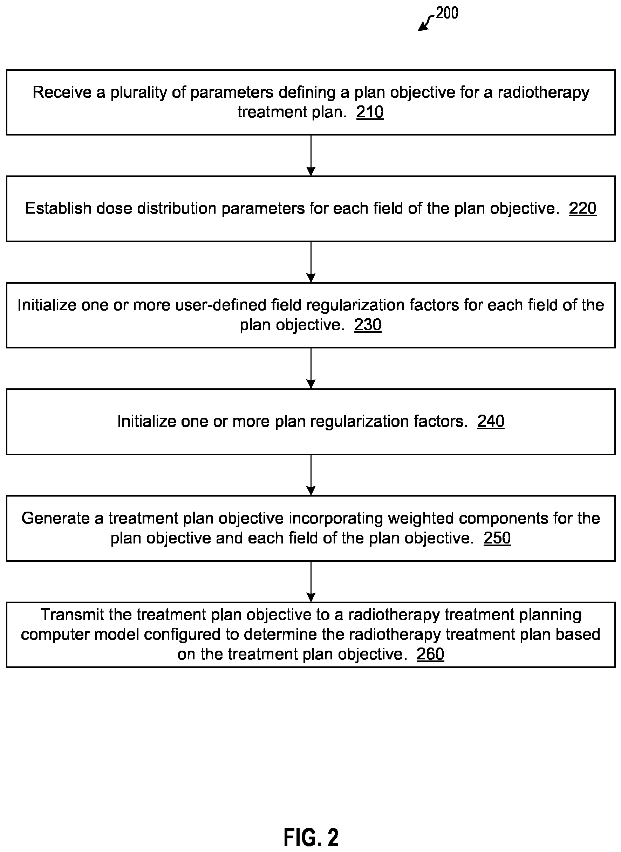

**FIG. 2** illustrates a flow diagram of a process executed in an AI-enabled RTTP generation system, according to an embodiment. The method **200** includes steps **210-260.** However, other embodiments may include additional or alternative execution steps or may omit one or more steps altogether. The method **200** describes how to control the amount of modulation in radiotherapy plans, such that the RTTP computer model can balance the conflicting objectives of achieving adequate dose conformity to the target volume while minimizing field modulation to reduce sensitivity to patient movement and improve treatment robustness. One or more steps of method **200** may be executed by any number of computing devices operating in the distributed computing system described in **FIG. 1****.** For instance, a different processor (or even a third-party processor) may train the artificial intelligence model compared to the processor executing the same artificial intelligence model. Moreover, the artificial intelligence model may be executed in conjunction with a plan optimizer that is being executed for and/or by a third party. Therefore, even though the method **200** is described as being executed/implemented by the analytics server, it is possible that each step of the method **200** is executed for a different entity/processor.

At step **210,** the analytics server may receive a plurality of parameters defining a plan objective for a radiotherapy treatment plan (RTTP). The radiotherapy treatment plan can be generated by a plan optimizer. The plan optimizer can be computer model(s) or software solution(s) that can run a plurality of simulations with various radiation or treatment parameters and can select, based on the simulation results, an initial set of parameters to be used. The analytics server may receive various treatment attributes determined by a treating physician or a clinician for a patient's radiotherapy treatment. These attributes may be determined for the patient based on the patient's treatment and are generally referred to as plan objectives. A non-limiting example of a plan objective may include dose limits, where a treating physician identifies the dosage that needs to be applied to the tumor. The analytics server may receive and/or retrieve (via a platform and/or via querying a database) patient data to generate a treatment plan for the patient. Therefore, as used herein, patient data may refer to any data that is associated with the patient (e.g., the patient's physical data, diagnosis data, and any data inputted by a medical professional). In some embodiments, the analytics server may use different patient identifiers to retrieve the patient data. For instance, the analytics server may query one or more databases to identify medical data associated with the patient. The analytics server may query data associated with the patient's anatomy, such as physical data (e.g., height, weight, and/or body mass index) and/or other health-related data (e.g., blood pressure or other data relevant to receiving radiation therapy treatment). The analytics server may also retrieve data associated with current and/or previous medical treatments received by the patient (e.g., data associated with the patient's previous surgeries).

In some embodiments, the analytics server may process the patient's medical data records to identify the patient attributes needed. For instance, the analytics server may query a database to identify the patient's body mass index (BMI). However, because many medical records are not digitized, the analytics server may not receive the patient's BMI value using simple query techniques. As a result, the analytics server may retrieve the patient's electronic health data and may execute one or more analytical protocols (e.g., natural language processing) to identify the patient's body mass index. In another example, if the analytics server does not receive tumor data (e.g., end-points), the analytics server may execute various image recognition protocols and identify the tumor data.

In some embodiments, the analytics server may receive additional data from one or more medical professionals. For instance, a treating oncologist may access a platform generated/hosted by the analytics server and may add, remove, or revise data associated with a particular patient, such as patient attributes, treatment attributes, tumor attributes, the primary site of treatment, tumor stage, endpoints, whether the primary tumor has been extended, and the like. Because tumor staging and the end-level attributes are critical pieces of information that affect patient treatment, this information may be inputted by the treating oncologist. In some embodiments, an AI model (e.g., a separate AI model that is trained to identify tumor information) may identify this information, and the treating oncologist may deny, approve, or revise the predicted results. In another example, the treating oncologist may specifically indicate whether the treatment should be unilateral or bilateral.

Another example of the plan objective and/or patient data/attributes may include specific tumor locations. More specifically, this data may indicate the primary tumor location with respect to the patient's centerline. This data may be inputted by the treating oncologist or may be analyzed using various image recognition or segmentation methods executed on the patient's medical images. In some embodiments, this information may be predicted using the AI model if it is not inputted by the treating oncologist (or otherwise received by the analytics server). Another patient attribute may indicate whether and how close the tumor volume (e.g., planning target volume) is to other non-diseased organs (e.g., organs at risk). For instance, a tumor to be eradicated may be millimeters away from another organ. This information may change field geometry (generated by the optimizer), as other organs must be avoided.

Based on the patient's data analysis and optimization process, the analytics server may generate the RTTP, for example, via the plan optimizer (or a radiotherapy treatment planning computer model) that may specify the number, angles, and shapes of the radiation beams used to deliver the treatment, the total dose of radiation to be delivered, and the fractionation schedule (number of treatment sessions and dose per session), among others.

In some embodiments, the analytics server may receive a plurality of parameters defining a plan objective for the RTTP. The plan objective may be comprised of several parameters, including, but not limited to, structure, volume, dose, and weight, among others. The structure may define the anatomical regions of interest, including the target tumor and organs at risk. The volume may specify the size or extent of the target volume and organs at risk. The dose may indicate the prescribed dose of radiation to be delivered to the target volume. The weight may assign priority or importance to different aspects of the plan objective. In some embodiments, additional parameters, such as dose constraints for organs at risk, dose uniformity metrics, or treatment time, may be included in the plan objective. The analytics server may use one or more parameters to generate the RTTP with the goal of precise dose delivery while minimizing potential adverse effects, such as those arising from patient movement.

At step **220,** the analytics server may establish dose distribution parameters for each field of the plan objective. In RTTP, the analytics server may establish the dose distribution parameters to deliver the prescribed dose of radiation to the target area while minimizing exposure to surrounding healthy tissues. In this regard, the analytics server may establish several dose distribution parameters, including, but not limited to, minimum dose, maximum dose, mean dose, dose-volume histogram (DVH), conformity index, and homogeneity index, among others. The analytics server may establish the minimum dose to ensure that all parts of the tumor receive at least the minimum required dose.

The analytics server may establish the maximum dose to avoid hot spots that may cause damage to healthy tissues. The analytics server may establish the mean dose to generate an overall assessment of the dose distribution. The DVH may illustrate a graphical representation that shows the distribution of dose within the target and surrounding tissues. The analytics server may establish the conformity index to determine how well the radiation dose conforms to the shape of the tumor volume. The analytics server may establish the homogeneity index to determine how uniformly the dose is distributed within the tumor volume.

In some embodiments, the analytics server may establish additional dose distribution parameters. For example, the analytics server may optimize each proton field individually to achieve a uniform dose over the target volume. In some embodiments, the analytics server may optimize the spot positions and weights of each proton field separately. In some embodiments, the analytics server may optimize all spots from all fields together, resulting in a highly conformal dose distribution. In some embodiments, the analytics server may simultaneously optimize the spots from multiple fields, which may lead to non-uniform dose distributions in individual fields but a more precise overall dose distribution.

In some embodiments, dose distribution parameters may include, but are not limited to, any combination of one or more of: ID/type, cubic centimeters, volume percentage, dose, actual dose, priority, and regions of interest (ROIs), among others. The ID/type may function as an identifier for each dose distribution parameter, specifying different objectives within the treatment plan. For example, ID/type values may include PTV (planning target volume), OAR (organ at risk), or other relevant anatomical regions. The cubic centimeters may provide a volume measurement unit for the target volume and organs at risk. The volume percentage may indicate the percentage of the target volume or organ covered by a specific dose level. The dose may measure the absorbed dose in gray (Gy) units, and the actual dose (also measured in Gy) may compare the delivered dose to the prescribed dose. In some embodiments, priority acts may be used as a weighting factor assigned to different dose objectives. The ROIs may refer to specific anatomical structures relevant to the RTTP.

In some embodiments, the analytics server may provide a control interface for users to adjust the dose distribution parameters. The interface may visually display the various dose distribution parameters. Users may modify parameter values through interactive elements such as sliders, text boxes, or dropdown menus, among others. In response to receiving an interaction with the interactive element, the analytics server may provide real-time or near-real-time visual feedback to show the impact of parameter changes on the RTTP.

In some embodiments, the analytics server may establish several fields in the RTTP to generate an effective RTTP. For example, the analytics server may establish any combination of one or more of: contours, beams, coverage, heterogeneity, OAR, and prescription, among other fields. The analytics server may outline the tumor volumes and OAR in the contours field to deliver the radiation dose precisely to the tumor volume. For beams, the analytics server may arrange and select the type of radiation beams used. For example, the analytics server may select simple (single or opposed fields) or complex (volumetric-modulated arc therapy, VMAT) beams to maximize tumor coverage and minimize exposure to healthy tissues. In the coverage field, the analytics server may determine how well the radiation dose covers the tumor volume to provide the entire tumor with the prescribed dose while minimizing the dose to surrounding healthy tissues.

The heterogeneity field may include the analytics server determining the uniformity of the radiation dose within the tumor volume, maintaining an even dose distribution, and avoiding hotspots and cold spots. For OAR the analytics server may set dose limits to prevent damage to healthy organs. In the prescription field, the analytics server may define the overall RTTP, including the total dose of radiation to be delivered, the number of treatment sessions (fractions), and/or the dose per session.

In some embodiments, the analytics server may establish additional fields, referring to the different angles or directions from which proton beams may be delivered to the tumor volume. The analytics server may use multiple beam angles to generate a highly conformal dose distribution around the tumor volume. The analytics server may optimize the intensities of proton beams from all fields simultaneously and balance the dose distribution across all fields to achieve the desired therapeutic effect. The analytics server may generate RTTP plans to be robust against uncertainties in patient positioning and proton range. In some embodiments, the analytics server may generate RTTP plans during the treatment course to account for anatomical changes in the patient, maintaining the effectiveness of the therapy throughout the treatment period.

At step **230,** the analytics server may initialize one or more user-defined field regularization factors for each field of the plan objective. Field regularization factors may indicate numerical values assigned to each radiation field in the RTTP. The analytics server may use the regularization factors to control the balance between achieving the field's specific goals (e.g., maximizing target coverage) and maintaining overall plan effectiveness (e.g., minimizing dose to organs at risk). For example, the optimizer or server may assign different (e.g. higher or lower) regularization factors to different fields. By assigning different (e.g. higher or lower) regularization factors to different fields, the optimizer can prioritize specific objectives, such as maximizing target coverage or minimizing OAR dose. The analytics server may apply the field regularization factors in various ways. For example, if the goal is to maximize target coverage, the analytics server may assign a higher regularization factor to the fields directly irradiating the tumor volume, thereby causing the optimizer to prioritize dose delivery to the target area. To protect sensitive organs at risk, the analytics server may assign lower regularization factors to fields that pass through or near these organs, thereby causing the optimizer to decrease dose delivery to these areas. In this regard, the analytics server may balance competing objectives, such as maximizing target coverage while minimizing OAR dose.

In some embodiments, the regularization factors may be associated with several parameters, including, but not limited to, any combination of one or more of: gantry rotation, monitor units (MU), beamline calculation, and beamline status, among others. The gantry rotation may refer to the angular position of the linear accelerator's gantry around the patient. The gantry may refer to a rotating arm of the linear accelerator (or linac) that positions the radiation beam. For example, the gantry rotation may be used to configure the angle at which radiation is delivered to the tumor volume. The MU may quantify the amount of radiation delivered by the linear accelerator and may be used to control the total dose delivered to the patient. The beamline calculation may define the path of the radiation beam from the linear accelerator to the patient, including the determination of the beam's shape, intensity distribution, and energy characteristics, among others. The beamline status may indicate the operational state of the beamline components, such as collimators, filters, and other devices that may affect the radiation beam.

In some embodiments, each regularization factor may be associated with multiple fields, which may be identified by respective field IDs (e.g., 1, 2, 3, etc.). The fields may correspond to specific operational parameters or settings within the RTTP. Each field may have a corresponding field value. For example, the gantry rotation may be indicated by multiple fields with field IDs 1, 2, and 3, each with a corresponding field value that specifies the angle or position of the gantry at different points in the treatment sequence. In some embodiments, the regularization factors may be applied to the corresponding field values to adjust their influence on the overall treatment process. The application of regularization can be configured according to the RTTP. The regularization, e.g. a regularization factor, may be quantified as a percentage value, which indicates the degree of influence that each field, or group of fields, has on the dose distribution. In some embodiments, the regularization, e.g. a regularization factor, may be applied individually to each field value. In other embodiments, the regularization, e.g. a regularization factor, may be applied collectively to all field values associated with a particular field ID.

In some embodiments, the analytics server may provide a control interface for users to adjust the regularization factor for the field objective(s) or the plan objective, depending on the implementation. The interface may visually display the regularization factor associated with each field objective, such as any combination of one or more of: gantry rotation, MU, beamline calculation, and beamline status, among other parameters. Users can adjust the regularization by interacting with various interactive elements, such as sliders, text boxes, or dropdown menus. In response to receiving an interaction with the interactive element, the analytics server may provide real-time or near-real-time feedback, via the control interface, to show the impact of regularization factor changes on the dose distribution. In some embodiments, the analytics server or the RTTP computer model may automatically adjust the regularization factor based on the tumor characteristics and other relevant factors.

In some embodiments, the analytics server may incorporate field-specific constraints, such as maximum or minimum dose limits for individual fields, using the regularization factors. In some embodiments, the analytics server may iteratively adjust the regularization factors as the RTTP evolves, for example, refining the balance between the various objectives to achieve a desired treatment outcome. In some embodiments, the analytics server may account for the tumor's location and size. For example, tumors near critical structures such as the brain or spinal cord may desire more precise targeting to avoid damaging these vital structures. The analytics server may configure different types of radiation beams and their energies based on the tumor's depth and type, for example, using higher energy beams for deeper tumors and lower energy beams for superficial tumors. In some embodiments, the analytics server may shape and modulate the radiation field using techniques, such as intensity-modulated radiation therapy (IMRT) and multileaf collimators (MLCs) to conform to the tumor's shape. In some embodiments, the analytics server may implement dose fractionation, for example, dividing the total radiation dose into smaller doses delivered over several sessions, depending on the tumor's radiosensitivity and the patient's overall health. In some embodiments, the analytics server can determine patient positioning and use immobilization devices to minimize movement such that the radiation targets the tumor precisely in each fraction. In some embodiments, the analytics server can use advanced imaging techniques such as computed tomography (CT), magnetic resonance imaging (MRI), and positron emission tomography (PET) to visualize the tumor and surrounding anatomy for precise treatment planning and validation.

At step **240,** the analytics server may initialize one or more plan regularization factors. The plan regularization factors may operate at the level of the entire RTTP to balance competing objectives across all fields, such as maximizing target coverage while minimizing dose to organs at risk. The plan regularization factors may refer to constraints applied during the RTTP to achieve a balance between delivering a sufficient dose to the tumor volume while minimizing exposure to surrounding organs at risk. The analytics server may use various types of plan regularization factors. For example, the analytics server may implement sparsity constraints to limit the number of active beam angles or source positions. In some embodiments, the analytics server may implement smoothness constraints to avoid abrupt changes in dose delivery. In some embodiments, the analytics server may implement dose constraints to keep the dose delivered to healthy organs at risk within safe limits.

In some embodiments, the plan regularization factors may be based on user-defined parameters or derived from the field regularization factors. For example, the plan regularization factors may be based on statistical aggregation, where the plan regularization factor is calculated as a statistical summary of the field regularization factors, such as the mean, median, or standard deviation. In some embodiments, the plan regularization factors may be based on a weighted combination, where the plan regularization factor is a weighted combination of field regularization factors. In some embodiments, the plan regularization factors may be directly linked to a specific field regularization factor or a combination of factors, generating a hierarchical relationship between plan and field levels. In some embodiments, the plan regularization factors may be defined independently by the user. The analytics server may use the plan regularization factors to optimize the overall treatment plan effectively.

At step **250,** the analytics server may generate a treatment plan objective incorporating weighted components for the plan objective and each field of the plan objective. In some embodiments, the analytics server may generate the treatment plan objective in response to applying the, e.g. user-defined, field regularization factor to a corresponding field of the plan objective and the plan regularization factor to the plan objective. The analytics server may adjust specific parameters for individual radiation fields, influencing aspects such as dose uniformity, gradient, and/or penumbra within each field, among others. For example, if a higher priority is given to maximizing dose uniformity within a field, the field regularization factor may be set accordingly. The analytics server may integrate constraints at the macro level to balance the contributions of all fields to achieve the overall treatment goals.

In some embodiments, upon establishing the field and plan regularization factors, the analytics server may combine the weighted components to determine the overall objective function value. The analytics server may determine the weighted component for each field by, for example, multiplying the corresponding field regularization factor by its field-specific objective. For instance, the field-specific objective may be calculated using parameters, such as dose uniformity, target coverage, and/or OAR sparing, etc. The analytics server may determine the weighted component of the plan objective by multiplying the plan regularization factor by the plan objective. The plan objective may incorporate parameters as described in connection with the field-specific objective or additional parameters, as described herein. The analytics server may combine the weighted components by, for example, summing the weight field component for all fields and adding the weight plan component to determine the resulting objective function value. The objective function value may indicate the overall performance of the RTTP based on the given parameters. In some embodiments, the plan objective may be scaled down by a regularization factor, for example, the user-defined field specific regularization factor, and assigned to individual field objectives within the treatment plan. Each field objective within the RTTP may receive the modified plan objective. The adjustment may be refined by the respective field-specific regularization factors such that the optimization of each field is aligned with the global treatment goals and localized considerations specific to each field.

At step **260,** the analytics server may transmit the treatment plan objective to a radiotherapy treatment planning computer model. The radiotherapy treatment planning computer model may determine the improved RTTP based on the treatment plan objective. In some embodiments, the RTTP computer model may utilize neural network models (plan optimizers, other machine learning models, or advanced algorithms) to automate various treatment planning tasks in response to processing the treatment plan objective. For example, the RTTP computer model may optimize beam geometry, intensity modulation, and dose distribution, among others, based on the provided treatment plan objective. In some embodiments, the RTTP computer model may identify that at least one structure (e.g., organs or tumors) of the patient experiences deviations (e.g., underdose or overdose) from the planned radiation dose. The deviation may refer to variations exceeding acceptable thresholds that may harm healthy organs or compromise tumor control, as defined by the treatment plan. For instance, if a tumor receives less radiation than planned, it may not shrink as expected.

In some embodiments, excessive radiation may cause inflammation of healthy organs. In some embodiments, patients may be evaluated against set schemas regarding side effects and/or known health-related issues. Non-limiting examples of these health-related issues may include xerostomia; headaches; hair loss; nausea; vomiting; extreme tiredness (fatigue); hearing loss or impairment; skin and/or scalp issues (changes), trouble with memory and/or speech, sore skin, diarrhea, fertility issues, and the like. When a health-related problem is identified, human reviewers (or sometimes using a processor that uses pre-set rules), may quantify the health-related problem, such that the quantified health-related problem and its corresponding category can be included within the training dataset for the computer models or machine learning models. Moreover, the health-related problem may include the development of secondary cancer. The training dataset may also include a timeline associated with each health-related problem.

In some embodiments, the RTTP computer model may predict a cost value that corresponds to the high-level utility of certain predicted probabilities, such as the probability of one or more side effects of other medical issues occurring. The cost value may correspond to a likelihood of the initial RTTP causing a health-related issue, such as long-term side effects or other health issues discussed herein. In some embodiments, the machine learning model (e.g., LSTM models) may be configured to calculate the cost value that can be directly used by the RTTP computer model. Additionally, or alternatively, the machine learning model may generate one or more scores that can then be used to calculate the cost value by the RTTP computer model. For instance, the values predicted by the machine learning model may be transformed (e.g., using other algorithms) to the cost value. Accordingly, the machine learning model can be trained to predict one or more attributes, which can then be converted into a cost value that is used by the RTTP computer model.

Based on the predictions, the RTTP computer model may recommend and/or generate a second radiotherapy treatment plan. For instance, in situations where significant and unexpected anatomical changes are predicted, a treatment replan may be generated. In some embodiments, the treatment replan may be automated, where the RTTP computer model automatically generates a new plan based on the predicted changes. In some embodiments, the treatment replan may include medical professionals reviewing available data, including scans and dose delivery information, to recalibrate or adjust doses (e.g., via a dose summation algorithm) for optimal treatment delivery despite the anatomical changes. For instance, the secondary dose deposition in the second treatment plan may differ from the initial dose deposition specified in the first treatment plan.

In cases of significant or unexpected anatomical changes, the RTTP computer model may automatically generate a revised treatment plan. This adaptive planning process may include recalculating dose distributions, adjusting beam parameters, or optimizing treatment delivery to account for the altered patient anatomy. Medical professionals may also be involved in reviewing the proposed treatment modifications and adjusting the RTTP accordingly.

In some embodiments, the RTTP computer model may implement the plan objective as MFO optimization, SFO optimization, or hybrid optimization, depending on the implementation. For example, when field regularization factors are minimized, the RTTP computer model may optimize the overall treatment plan without prioritizing individual fields. In some embodiments, when both field and plan regularization factors are maximized, the RTTP computer model may optimize each field independently to achieve maximum target coverage. For intermediate values of regularization factors, the RTTP computer model may combine elements of MFO and SFO to balance treatment goals. In some embodiments, the RTTP can parse the plan objective into field-specific objectives. The RTTP can optimize each field objective based on the respective specific constraints, such as maximizing target volume while minimizing OAR dose.

In a non-limiting example of the implementation of the methods and systems discussed herein, a patient is being treated for a tumor located in the abdomen. The goal is to deliver a uniform dose of radiation to the tumor while minimizing exposure to surrounding healthy tissues and OARs (e.g., the liver and kidneys). Traditionally, a treatment plan might use two or more radiation fields (beams) aimed at the tumor from different angles. The optimization algorithm ensures that the total dose delivered by all fields combined meets the prescribed dose for the tumor. However, using these traditional approaches can lead to uneven dose distributions within each individual field. For instance, one field might deliver a higher dose to one side of the tumor and a lower dose to the other, while the opposite field might have the high and low dose regions flipped. When these fields are combined, the overall dose to the tumor is uniform, but individually, the fields have significant "hot spots" (areas of higher dose) and "cold spots" (areas of lower dose).

Using the method **200,** the treatment planner can address this issue by establishing a dose distribution parameter, known as the regularization parameter. In this scenario, the planner might set the regularization parameter to 20%, meaning the algorithm will focus 80% on the overall plan objectives and 20% on achieving uniform dose distribution within each field. The optimization process then begins, considering both the overall plan objectives-such as delivering the prescribed dose to the tumor and sparing organs at risk-and the individual field objectives. With the regularization parameter set at 20%, the algorithm slightly adjusts the dose distributions within each field to make them more even, while still ensuring that the combined dose from all fields meets the overall plan objectives.

As a result of this adjustment, each field may have a more balanced dose distribution, reducing the extreme hot and cold spots that might have occurred without this regularization. The combined dose across all fields still meets the prescribed dose for the tumor, and the dose to organs at risk is kept within acceptable limits. This more uniform dose distribution within each field reduces the risk of "hot spots" and "cold spots" if the patient moves slightly between the delivery of different fields. The overall treatment plan is thus more robust, ensuring that the tumor receives the correct dose even in the presence of minor patient movement or other uncertainties during treatment.

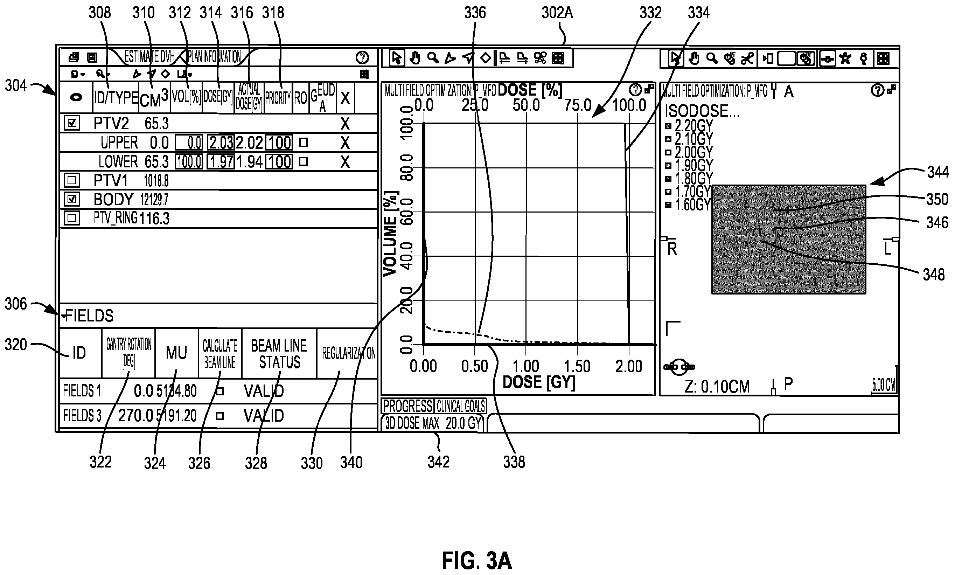

Referring now to **FIG. 3A****,** depicted is an example user interface illustrating an MFO plan. As shown on the top left of a graphical user interface **302A,** the graphical user interface **302A** may display details about dose distribution parameters **304** and fields **306.** The dose distribution parameters **304** and field **306** may be presented in column or row layouts, depending on the implementation. The dose distribution parameters **304** may include ID/type **308,** cm³ **310,** vol (%) **312,** dose (Gy) **314,** actual dose (Gy) **316,** and priority **318,** among others. The ID/type **308** may identify the structure or objectives, such as PTV2, PTV1, Body, or PTV_Ring. The cm³ **310** may indicate the volume of the structure in cubic centimeters. The Vol (%) **312** may indicate the percentage of the structure that receives a dose, for example, greater than or equal to the specified dose. The dose (GY) **314** may indicate the dose in gray units. The Actual dose **316** may indicate the actual dose delivered to the structure. The priority **318** may indicate the priority of the structure in the treatment.

On the bottom left of the graphical user interface **302A,** the graphical user interface **302A** may display details about fields **306** used in the treatment plan. There may be one or more fields **306,** depending on the implementation. Each field **306** may include ID **320,** gantry rotation (deg) **322,** monitor units (MU) **324,** calculate beamline **326,** beam line status **328,** and regularization **330,** among others. The ID **320** may correspond to the associated field **306.** The gantry rotation **322** may display the angle of the gantry for the corresponding field **306.** The MU **324** may indicate the monitor units for the corresponding field **306.** The calculate beamline **326** may indicate whether the beamline has been calculated for the corresponding field **306.** The beam line status **328** may indicate the status of the beamline for the corresponding field **306.** For example, the beam line status **328** may indicate whether the status of the field is valid or invalid. The regularization **330** may indicate the degree of influence of the corresponding field **306** on the overall treatment plan. The regularization **330** may be expressed as a numerical value or a percentage.

In the middle of the graphical user interface **302A,** the graphical user interface **302A** may display a dose-volume histogram (DVH) plot **332.** The DVH plot **332** may indicate the distribution of radiation doses. In this example, the DVH plot **332** may display two lines representing the dose distribution for PTV2 and Body. The solid line **334** may correspond to a type **308** of structure or objective, such as planning target volume (PTV 2), indicating the volume of the target region receiving different dose levels. The dotted line **336** may also correspond to a type **308** of structure or objective, such as Body, indicating the volume of healthy tissue exposed to various radiation doses. The x-axis **338** of the DVH plot **332** may indicate the radiation dose in Gy units. For example, the radiation dose may range from 0 Gy (no dose) to 2 Gy. The y-axis **340** of the DVH plot **332** may indicate the volume of the structure in percentage. For example, the volume may range from 0% (no volume) to 100% (total volume) of the structure. In some implementations, the graphical user interface **302A** may display the maximum radiation dose **342** delivered at any point in the treatment volume. The DVH plot **332** can be configured to provide information about the dose distribution within the target volume (PTV2) and the surrounding healthy tissue (body). For example, as shown, a steep slope in the PTV line **334** at higher dose levels may indicate adequate target coverage, and a flatter curve for the body line **336** at lower dose levels may indicate reduced exposure of healthy tissues.

On the right side of the graphical user interface **302A,** the graphical user interface 302A may display an isodose distribution plot **344** to provide a visual representation of a radiation treatment plan. The isodose distribution plot **344** may provide the distribution of radiation dose within a specific cross-sectional plane of the patient. The isodose distribution plot **344** may include multiple contour lines **346** to indicate areas of equal radiation dose. The contour lines **346** may be labeled with their corresponding dose values in Gy units. The dose values may range from 1.60 Gy to 2.20 Gy. In some embodiments, higher isodose levels may correspond to regions receiving higher radiation doses. The graphical user interface **302A** may be configured to display a target region **348.** The graphical user interface **302A** may display the target region **348** as a circular or oval-shaped region within the isodose distribution to indicate the target tumor or treatment area. The target region **348** may indicate the concentration of radiation dose in the desired area. The surrounding area **350** outside the target region **348** may indicate healthy tissue.

The isodose curves in the surrounding area **350** may indicate the distribution of radiation doses in the surrounding tissues. In some embodiments, the isodose distribution plot **344** may include a grid for spatial reference and labels indicating the orientation (e.g., R for right, L for left, A for anterior, P for posterior). In some embodiments, the isodose distribution plot **344** may include the z-coordinate to indicate the depth of the cross-sectional plane.

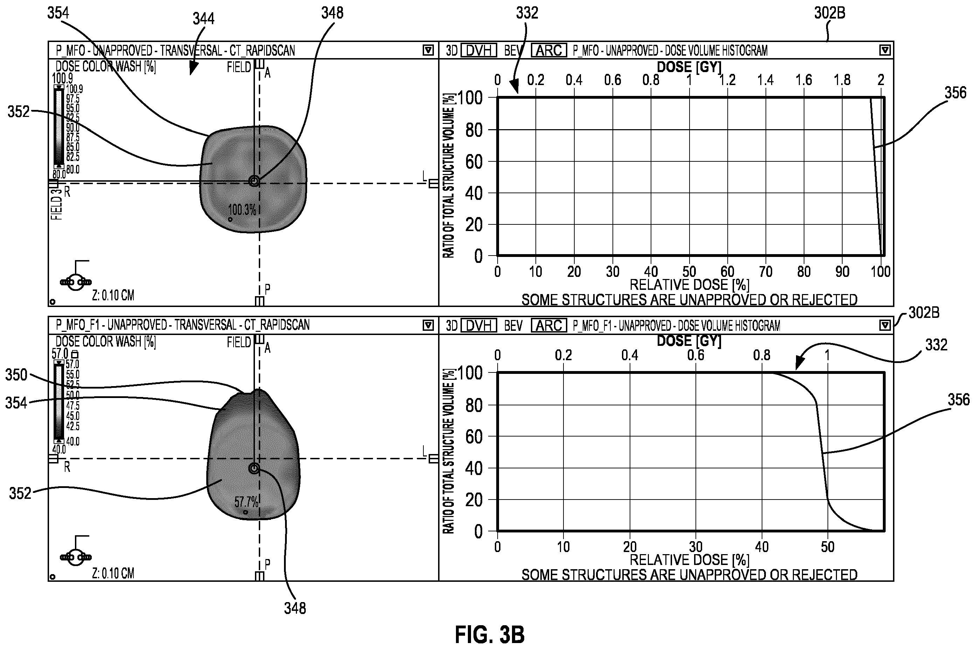

Referring now to **FIG. 3B****,** depicted is an example comparison of the overall plan dose with the dose distribution of individual fields. For example, **FIG. 3B** depicts an issue in intensity-modulated radiation therapy (IMRT) planning, where the optimization process may lead to highly modulated field doses. While the overall plan dose may appear satisfactory, individual field doses may show significant variations.

The upper portion of the graphical user interface **302B** displays the overall plan dose distribution. As shown, the upper portion of the graphical user interface **302B** displays the isodose distribution plot **344** and the DVH plot **332,** as described in connection with **FIG. 3A****.** The upper portion of the graphical user interface **302B** shows a smooth and uniform distribution of radiation dose across the target region **348.** A central, darker region **352** within the isodose distribution plot **344,** referred to as a hot spot, indicates an area of elevated dose. Surrounding the hotspot **352** is a cold spot **354** with lower dose levels. The cold spot **354** can indicate an area within the treatment plan that receives a lower dose of radiation.

The lower portion of the graphical user interface **302B** displays the dose distribution for an individual treatment field **306.** In contrast to the smooth plan dose distribution, the field dose can be seen as exhibiting a high degree of modulation, with areas of high and low dose intensity. This phenomenon, also referred to as the jigsaw effect, may arise from the desire to combine multiple fields with complementary dose distributions to achieve a uniform overall plan. The cold spots **354** observed in the individual field dose distribution may extend into the healthy surrounding area **350.** The hot spot **352** in the individual field dose distribution is not as concentrated as it should be, indicating that the dose distribution is not effectively targeting the intended target region **348.** This dispersion may lead to less effective treatment of the tumor and potentially greater exposure of healthy tissues to higher radiation doses. The individual field doses may be sensitive to patient movement, leading to unintended hot spots **352** and cold spots **354** in the patient if the patient shifts position between field deliveries.

The DVH plot **332** in the upper portion of the graphical user interface **302B** displays a characteristic curve **356.** As shown, the curve **356** remains relatively flat at 100 volume-% from 0 Gy to approximately 1.9 Gy, after which it steeply descends to 0 volume-% between 1.9 Gy and 2.0 Gy. This behavior indicates that a significant portion of the target volume receives a uniform dose. The lower portion of the graphical user interface **302B** displays the DVH plot **332** for an individual treatment field **306.** Unlike the upper plot, the lower plot often displays a less steeply descending DVH slope, indicating a less homogeneous dose for the target structure. This non-uniform dose distribution within the field **306** may lead to areas of higher and lower dose concentration, impacting treatment efficacy and increasing the risk of side effects.

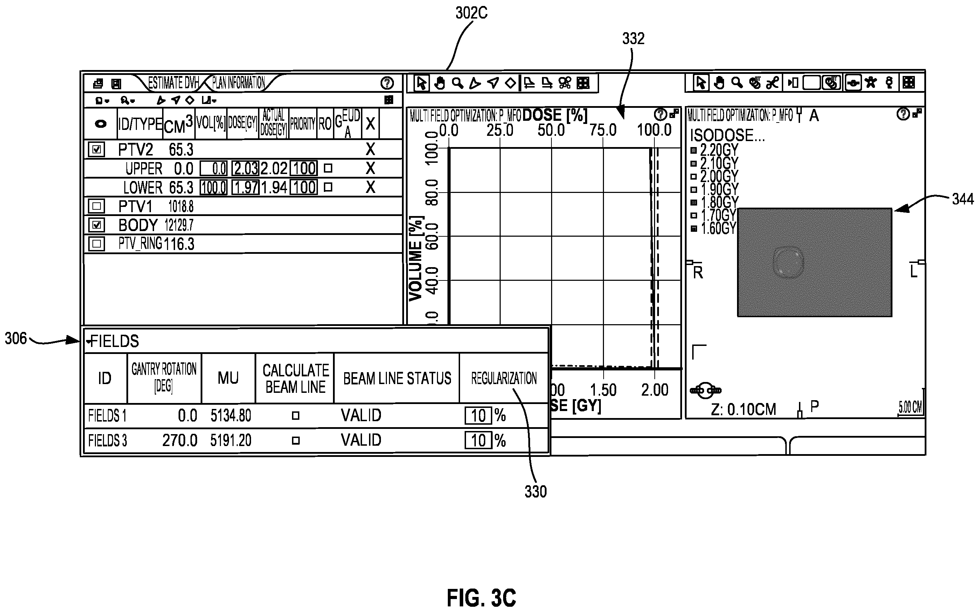

Referring now to **FIG. 3C****,** depicted is an example user interface allowing for the manipulation of regularization parameters to control the degree of modulation in a multi-field optimized (MFO) treatment plan, as described in connection with **FIG. 3A****.** A user can select specific fields **306** to view the corresponding isodose distribution plot **344** and the DVH plot **332** in the graphical user interface **302C.** The user can modify parameters by interacting with the regularization **330** (e.g., displayed as a user-controllable element) to control the balance between plan-level optimization and field-level uniformity. In the illustrated example, the regularization **330** is set to 10% for fields 1 and 3. The configuration may indicate a moderate level of regularization, for example, aiming to balance the conflicting objectives of target coverage and dose uniformity. In some embodiments, a higher regularization value may prioritize field-level uniformity at the expense of overall plan quality. In some embodiments, a lower regularization value may allow for greater flexibility in field shaping but may increase the risk of dose heterogeneity. The graphical user interface **302C** can allow for iterative adjustments of the regularization **330** to achieve the desired balance between plan-level objectives and field-level uniformity. For example, by determining the impact of regularization changes on dose distribution and plan metrics, clinicians can fine-tune the treatment plan to optimize patient outcomes.

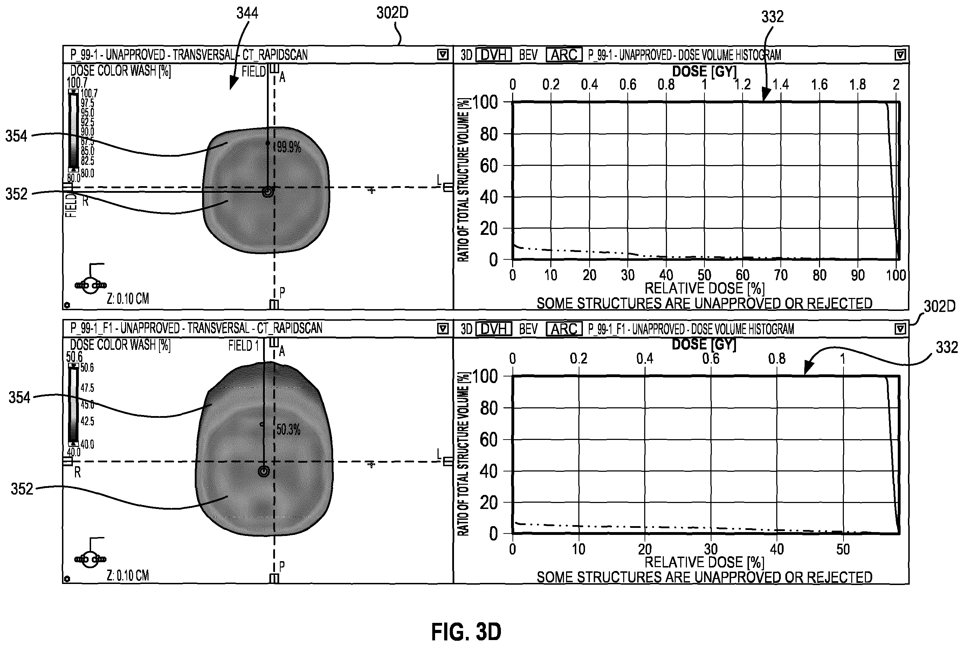

Referring now to **FIG. 3D****,** depicted is an example user interface illustrating the effects of applying a regularization parameter on the dose distribution of a treatment plan. **FIG. 3D** demonstrates the impact of regularization **330** (e.g., 10% regularization) on both the overall plan dose and individual field doses. The upper portion of the graphical user interface **302D,** displaying the overall plan dose distribution, remains largely unchanged, indicating that the application of regularization **330** has not significantly compromised the primary treatment objectives. The target coverage and sparing of healthy tissues, as indicated by the plan dose distribution (including the DVH plot **332** and isodose distribution plot **344),** remain consistent before and after the application of regularization **330.**

However, a notable difference may be observed in the lower portion of the graphical user interface **302D,** which depicts the individual field dose distribution (including the DVH plot **332** and isodose distribution plot **344).** The application of regularization **330** has led to a reduction in the degree of modulation within the individual fields, mitigating the jigsaw effect observed in FIG. **3B****.** For example, the hot spot **352,** previously less concentrated and extending beyond the target region **348,** is now more focused within the target area **348** in **FIG. 3D****.** Similarly, the cold spots **354,** which extended into healthy tissues in the surrounding area **350** in **FIG. 3B****,** are reduced in size and intensity in **FIG. 3D****.**

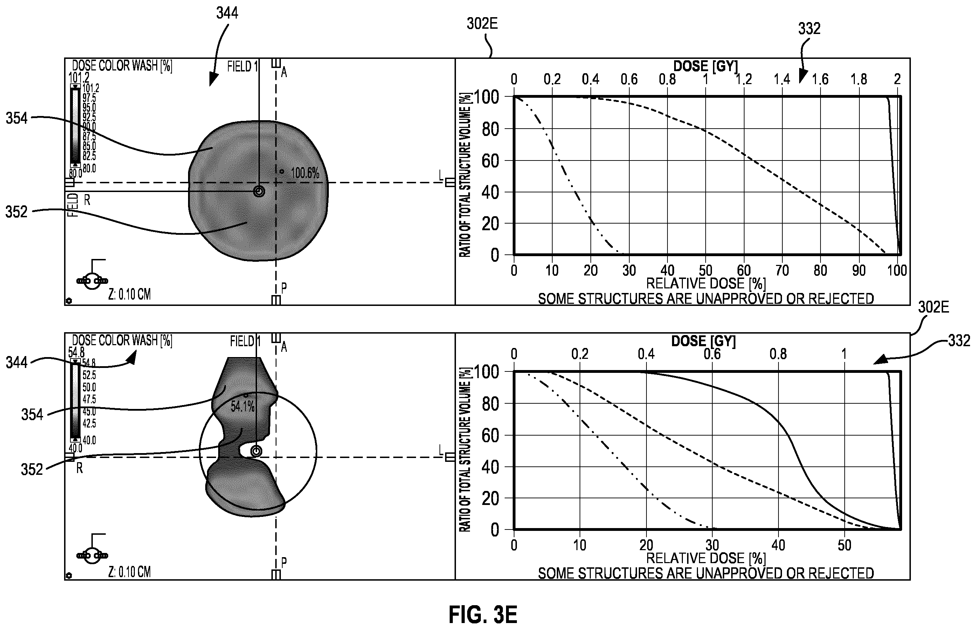

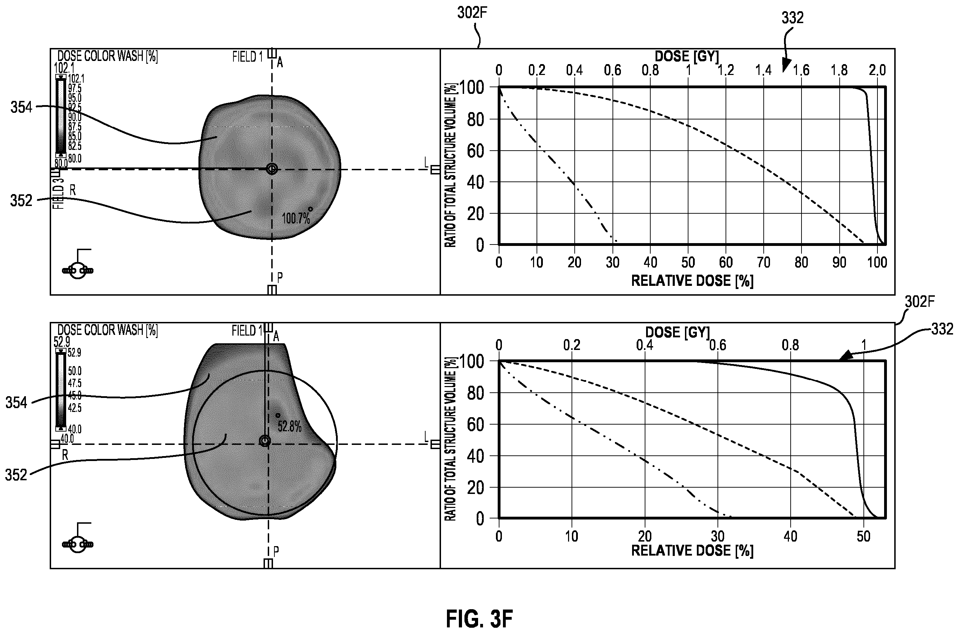

Referring now to **FIGS. 3E-3F****,** depicted is an example user interface illustrating an MFO plan with the additional objective of OAR sparing. The graphical user interface **302E** in **FIG. 3E** displays an MFO plan where a degree of modulation is introduced to achieve acceptable OAR sparing while maintaining adequate PTV coverage. While the overall plan dose appears satisfactory, individual field doses exhibit significant inhomogeneity. The graphical user interface **302F** in **FIG. 3F** illustrates the impact of applying a regularization parameter **330** of 10% to the plan. The regularization process effectively reduces field-level modulation while preserving overall plan quality, including target coverage and OAR sparing. The hot spots **352** and cold spots **354** observed in the individual field dose distribution of **FIG. 3E** are significantly reduced in **FIG. 3F****,** indicating the effectiveness of the regularization technique in generating a more uniform dose distribution within each field. For example, the hot spots **352,** prominent in **FIG. 3E** and extending beyond the target region **348,** are significantly reduced and more centrally located in **FIG. 3F****.** Similarly, the cold spots **354** observed in **FIG. 3E** are less pronounced in **FIG. 3F****,** indicating a more uniform dose distribution within the individual fields.

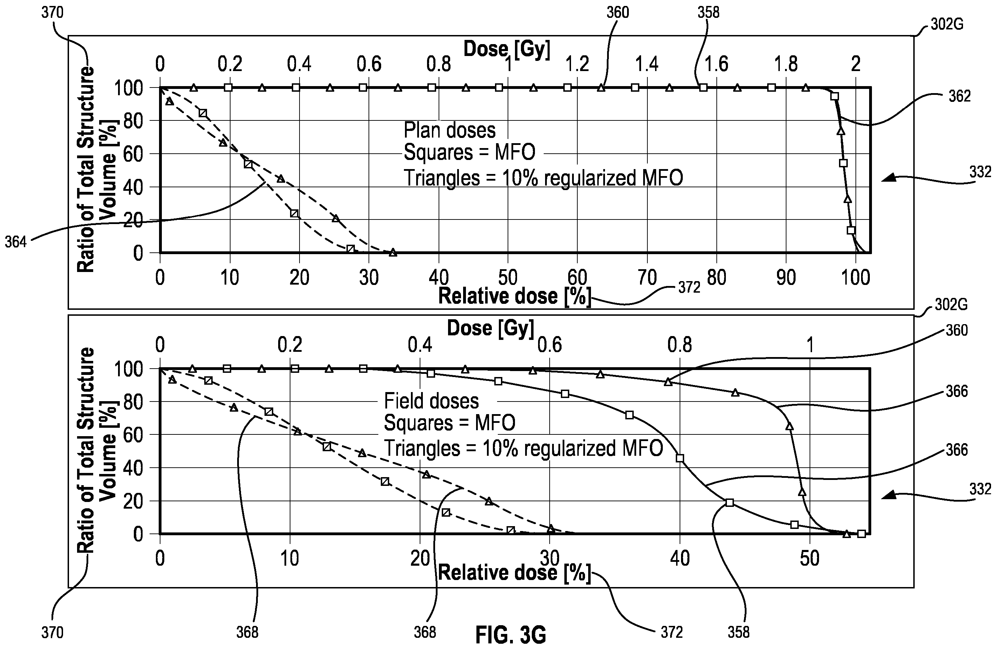

Referring now to **FIG. 3G****,** depicted is an example user interface illustrating the DVH plots **332** for the plan dose and individual field doses. The upper portion of the graphical user interface **302G** displays the DVH plot **332** for the overall plan. The lower portion of the graphical interface **302G** displays the DVH plot **332** for an individual field. In both plots, a square line **358** indicates the dosage data for the standard MFO optimized plan, and a triangle line **360** indicates the dosage data for the 10% regularized MFO optimized plan. For the plan dose DVH plot **332,** a solid line **362** indicates the plan dose distribution, and a dotted line **364** indicates the OAR dose. For the field dose DVH plot **332,** the solid line **366** indicates the field dose, and the dotted line **368** indicates the corresponding OAR dose. The y-axis **370** of both DVH plots **332** indicates the percentage of the total volume receiving a dose greater than or equal to the dose on the x-axis **372,** which indicates the relative dose in percentage.

**FIG. 3G** can allow for a direct comparison of the dose distribution between the standard and regularized MFO plans. For example, the overall plan DVH plot **332** shows minimal differences between the two optimization methods. However, the field-level DVH plot **332** clearly demonstrates the impact of regularization on reducing dose heterogeneity. For example, a more gradual slope in the square line **358** (indicating the standardized MFO plan) shows a higher degree of dose heterogeneity. The 10% regularized MFO line has a steeper slope in the triangle line **360.** This shows that the dose is spread out more evenly, and there are fewer differences in dose within the treated volume.

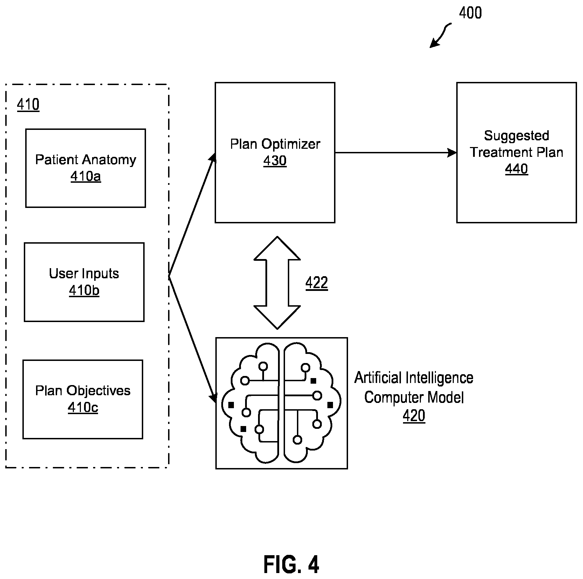

Referring now to **FIG. 4****,** a non-limiting visual example of a workflow utilizing the methods and systems described herein is illustrated. In this non-limiting example **400,** the analytics server provides plan objectives **410c,** patient anatomy **410a,** user inputs **410b,** and other data that may be needed to generate an RTTP (collectively patient data **410)** to the plan optimizer **430** to generate a suggested RTTP that is optimized for a patient and their treatment. The analytics server may also transmit the data **410** to the artificial intelligence computer model **420.** As a result, the artificial intelligence computer model **420** may transmit a predicted treatment plan objective, prioritizing dose conformity while minimizing field modulation. The artificial intelligence computer model **420** may transmit the predicted treatment plan objective to the plan optimizer **430,** where the two models can iteratively work together to generate and/or revise the suggested treatment plan **440.**

The analytics server may first collect patient data **410.** The patient data may include patient anatomy data **410a** (e.g., medical images, PTVs, OARs), and user inputs **410b** (clinical objectives, regularization parameters, or rules received via a user interface from a treating oncologist, such as tumor data, PTV identification, and the like). Other non-limiting examples of clinical goals may include dosimetric goodness function, robustness metrics, biological effects of radiation, metrics of linear energy transfer, and the like. The patient data **410** may also include rules **410c** for the patient's treatment (e.g., clinical/treatment objectives or criteria identified by the medical professionals or any other special treatments required by the medical professionals).

In some configurations, the analytics server may access a patient's internal/external file and retrieve/extract the needed patient data **410.** The analytics server may then execute an artificial intelligence computer model **420** to identify a cost function for a candidate RTTP for the patient using the patient data **410.** The results generated via the artificial intelligence computer model **420** may be ingested by the plan optimizer **430.** The plan optimizer **430** may be a treatment planning and/or monitoring software solution. The plan optimizer **430** may analyze various factors associated with the patient and the patient's treatment to generate and optimize an RTTP for the patient (e.g., field geometry, treatment modality, and radiation and/or treatment parameters needed to treat the patient).

One of the factors considered by the plan optimizer **430** may be the cost value calculated by the artificial intelligence computer model **420.** The plan optimizer **430** may iteratively revise the patient's RTTP, wherein the plan optimizer **430** iteratively revises different attributes of the RTTP (e.g., dose constraints delivered to the planning target volume and organs at risk, field geometry, etc.). In some configurations, the plan optimizer **430** may transmit new treatment plan data back to the artificial intelligence model **420,** whereby the dose calculation sector model **420** can recalculate/re-predict the cost value based on the revised treatment data generated by the plan optimizer (iteration **422).** The plan optimizer **430** and the artificial intelligence model **420** may repeat iteration **422** until the patient's RTTP is optimized/finalized.

When the plan optimizer **430** completes the patient's RTTP, the plan optimizer **430** may transmit the suggested treatment plan **440** to one or more electronic devices where a user (e.g., clinician) can review the suggested plan. For instance, the suggested treatment plan **440** (or any attributes predicted by the dose calculation sector model **420)** may be displayed on a computer of a clinic where a radiation therapy technician or a treating oncologist can review the data.

The various illustrative logical blocks, modules, circuits, and algorithm steps described in connection with the embodiments disclosed herein may be implemented as electronic hardware, computer software, or combinations of both. To clearly illustrate this interchangeability of hardware and software, various illustrative components, blocks, modules, circuits, and steps have been described above generally in terms of their functionality. Whether such functionality is implemented as hardware or software depends upon the particular application and design constraints imposed on the overall system. Skilled artisans may implement the described functionality in varying ways for each particular application, but such implementation decisions should not be interpreted as causing a departure from the scope of this disclosure or the claims.

Embodiments implemented in computer software may be implemented in software, firmware, middleware, microcode, hardware description languages, or any combination thereof. A code segment or machine-executable instructions may represent a procedure, a function, a subprogram, a program, a routine, a subroutine, a module, a software package, a class, or any combination of instructions, data structures, or program statements. A code segment may be coupled to another code segment or a hardware circuit by passing and/or receiving information, data, arguments, parameters, or memory contents. Information, arguments, parameters, data, etc. may be passed, forwarded, or transmitted via any suitable means including memory sharing, message passing, token passing, network transmission, etc.

The actual software code or specialized control hardware used to implement these systems and methods is not limiting of the claimed features or this disclosure. Thus, the operation and behavior of the systems and methods were described without reference to the specific software code being understood that software and control hardware can be designed to implement the systems and methods based on the description herein.

When implemented in software, the functions may be stored as one or more instructions or codes on a non-transitory computer-readable or processor-readable storage medium. The steps of a method or algorithm disclosed herein may be embodied in a processor-executable software module, which may reside on a computer-readable or processor-readable storage medium. A non-transitory computer-readable or processor-readable media includes both computer storage media and tangible storage media that facilitate the transfer of a computer program from one place to another. A non-transitory processor-readable storage media may be any available media that may be accessed by a computer. By way of example, and not limitation, such non-transitory processor-readable media may comprise RAM, ROM, EEPROM, CD-ROM or other optical disk storage, magnetic disk storage or other magnetic storage devices, or any other tangible storage medium that may be used to store desired program code in the form of instructions or data structures and that may be accessed by a computer or processor. Disk and disc, as used herein, include compact disc (CD), laser disc, optical disc, digital versatile disc (DVD), floppy disk, and Blu-ray disc where disks usually reproduce data magnetically, while discs reproduce data optically with lasers. Combinations of the above should also be included within the scope of computer-readable media. Additionally, the operations of a method or algorithm may reside as one or any combination or set of codes and/or instructions on a non-transitory processor-readable medium and/or computer-readable medium, which may be incorporated into a computer program product.

The preceding description of the disclosed embodiments is provided to enable any person skilled in the art to make or use the embodiments described herein and variations thereof. Various modifications to these embodiments will be readily apparent to those skilled in the art, and the principles defined herein may be applied to other embodiments without departing from the scope of the subject matter disclosed herein. Thus, the present disclosure is not intended to be limited to the embodiments shown herein but is to be accorded the widest scope consistent with the following claims and the principles and novel features disclosed herein.

While various aspects and embodiments have been disclosed, other aspects and embodiments are contemplated. The various aspects and embodiments disclosed are for purposes of illustration and are not intended to be limiting, with the true scope being indicated by the following claims.

## Claims

1. A system comprising:
one or more processors configured to:
receive a plurality of parameters defining a plan objective for a radiotherapy treatment plan;
establish dose distribution parameters for each field of the plan objective;
initialize one or more user-defined field regularization factors for each field of the plan objective;
initialize one or more plan regularization factors, wherein the plan regularization factor is based on at least one of the user-defined field regularization factor or user-defined parameters;
in response to applying the user-defined field regularization factor to a corresponding field of the plan objective and the plan regularization factor to the plan objective, generate a treatment plan objective incorporating weighted components for the plan objective and each field of the plan objective; and
transmit the treatment plan objective to a radiotherapy treatment planning computer model, the radiotherapy treatment planning computer model configured to determine the radiotherapy treatment plan based on the treatment plan objective.

2. The system of claim 1, wherein the plurality of parameters include at least one of a structure, dose, volume, or weight.

3. The system of claim 1 or claim 2, wherein the one or more processors are further configured to initialize values for the plan regularization factor and the user-defined field regularization factor.

4. The system of any preceding claim, wherein the one or more processors are further configured to calculate the weighted component for the plan objective in response to applying the plan regularization factor to the plan objective.

5. The system of any preceding claim, wherein the one or more processors are further configured to calculate the weighted component for the corresponding field of the plan objective in response to applying the user-defined field regularization factor to the corresponding field of the plan objective.

6. The system of any preceding claim, wherein the one or more processors are further configured to provide a control interface configured to receive user interactions for adjusting the user-defined regularization factors.

7. The system of any preceding claim, wherein the one or more processors are further configured to implement the plan objective as a multi-field optimization formulation responsive to identifying that the user-defined field regularization factors are set to a value of zero;
and/or:
wherein the one or more processors are further configured to implement the plan objective as a full single field optimization coverage of planning target volume responsive to identifying that the user-defined field regularization factors and the plan regularization factor are set to a value of one.

8. A method for radiotherapy treatment plan generation, the method comprising:
receiving, by at least one processor, a plurality of parameters defining a plan objective for a radiotherapy treatment plan;
establishing, by the at least one processor, dose distribution parameters for each field of the plan objective;
initializing, by the at least one processor, one or more user-defined field regularization factors for each field of the plan objective;
initializing, by the at least one processor, one or more plan regularization factors, wherein the plan regularization factor is based on at least one of the user-defined field regularization factor or user-defined parameters;
in response to applying the user-defined field regularization factor to a corresponding field of the plan objective and the plan regularization factor to the plan objective, generating, by the at least one processor, a treatment plan objective incorporating weighted components for the plan objective and each field of the plan objective; and
transmitting, by the at least one processor, the treatment plan objective to a radiotherapy treatment planning computer model, the radiotherapy treatment planning computer model configured to determine the radiotherapy treatment plan based on the treatment plan objective.

9. The method of claim 8, wherein the plurality of parameters include at least one of a structure, dose, volume, or weight.

10. The method of claim 8 or claim 9, further comprising initializing, by the at least one processor, values for the plan regularization factor and the user-defined field regularization factor;
and/or:
the method further comprising calculating, by the at least one processor, the weighted component for the plan objective in response to applying the plan regularization factor to the plan objective.

11. The method of any of claim 8 to 10, further comprising, calculating, by the at least one processor, the weighted component for the corresponding field of the plan objective in response to applying the user-defined field regularization factor to the corresponding field of the plan objective;
and/or:
the method further comprising providing, by the least one processor, a control interface configured to receive user interactions for adjusting the user-defined regularization factors.

12. A non-transitory machine-readable storage medium having computer-executable instructions stored thereon that, when executed by one or more processors, cause the one or more processors to:
receive a plurality of parameters defining a plan objective for a radiotherapy treatment plan;
establish dose distribution parameters for each field of the plan objective;
initialize one or more user-defined field regularization factors for each field of the plan objective;
initialize one or more plan regularization factors, wherein the plan regularization factor is based on at least one of the user-defined field regularization factor or user-defined parameters;
in response to applying the user-defined field regularization factor to a corresponding field of the plan objective and the plan regularization factor to the plan objective, generate a treatment plan objective incorporating weighted components for the plan objective and each field of the plan objective; and
transmit the treatment plan objective to a radiotherapy treatment planning computer model, the radiotherapy treatment planning computer model configured to determine the radiotherapy treatment plan based on the treatment plan objective.

13. The non-transitory machine-readable storage medium of claim 12, wherein the plurality of parameters include at least one of a structure, dose, volume, or weight.

14. The non-transitory machine-readable storage medium of claim 12 or claim 13, wherein the instructions stored thereon that, when executed by the one or more processors, cause the one or more processors to initialize values for the plan regularization factor and the user-defined field regularization factor;
and/or:
wherein the instructions stored thereon that, when executed by the one or more processors, cause the one or more processors to calculate the weighted component for the plan objective in response to applying the plan regularization factor to the plan objective.

15. The non-transitory machine-readable storage medium of any of claim 12 to claim 14, wherein the instructions stored thereon that, when executed by the one or more processors, cause the one or more processors to calculate the weighted component for the corresponding field of the plan objective in response to applying the user-defined field regularization factor to the corresponding field of the plan objective;
and/or:
wherein the instructions stored thereon that, when executed by the one or more processors, cause the one or more processors to provide a control interface configured to receive user interactions for adjusting the user-defined regularization factors.
